(19) Europäisches Patentamt
European Patent Office
Office européen des brevets

(11) **EP 0 783 902 B1**

(12) **EUROPÄISCHE PATENTSCHRIFT**

(45) Veröffentlichungstag und Bekanntmachung des Hinweises auf die Patenterteilung:
**26.10.2005 Patentblatt 2005/43**

(51) Int Cl.⁷: **A61N 1/372**

(21) Anmeldenummer: **96250292.8**

(22) Anmeldetag: **17.12.1996**

(54) **Extrakorporales Kontrollgerät für ein implantierbares medizinisches Gerät**

Extracorporal control device for an implantable medical device

Dispositif de contrôle extracorporel pour un dispositif médical implantable

(84) Benannte Vertragsstaaten:
**DE FR GB IT NL SE**

(30) Priorität: **18.12.1995 DE 19548658**

(43) Veröffentlichungstag der Anmeldung:
**16.07.1997 Patentblatt 1997/29**

(73) Patentinhaber: **BIOTRONIK Mess- und Therapiegeräte GmbH & Co Ingenieurbüro Berlin**
**12359 Berlin (DE)**

(72) Erfinder: **Dietrich, Michael**
**12161 Berlin (DE)**

(74) Vertreter: **von Oppen, Joachim F.M.**
**Eisenführ, Speiser & Partner**
**Anna-Louisa-Karsch-Strasse 2**
**10178 Berlin (DE)**

(56) Entgegenhaltungen:
**DE-A- 4 417 927       DE-C- 3 440 615**
**US-A- 4 862 449       US-A- 5 113 869**
**US-A- 5 357 969       US-A- 5 421 830**

**Beschreibung**

Die Erfindung betrifft eine extrakorporale Vorrichtung zur Benutzung mit einem implantierbaren medizinischen Gerät, insbesondere einem Herzschrittmacher, Defibrillator oder Kardioverter, welches zur Aufnahme und Verarbeitung eines, insbesondere die Funktion des implantierbaren Geräts beeinflussenden, über einen intrakorporalen Signalaufnehmer aufgenommenen Meßsignals einen Verarbeitungskanal aufweist, in dem ein erstes und ein zweites signalbeeinflussendes Übertragungsglied vorgesehen ist, wobei zur Übertragung eines von dem Meßsignal abgeleiteten Telemetriesignals an das Kontrollgerät eine erste Telemetrieeinheit vorgesehen ist, deren Eingang mit dem Ausgang des ersten Übertragungsglieds und dem Eingang des zweiten Übertragungsglieds verbunden ist.

Die extrakorporale Vorrichtung umfasst weiter eine zweite Telemetrieeinheit zum Empfang des von dem implantierbaren Gerät ausgesandten Telemetriesignals und ein der zweiten Telemetrieeinheit nachgeschaltetes drittes Übertragungsglied zur Kompensation der in dem Telemetriesignal enthaltenen, durch das erste Übertragungsglied bewirkten Signalbeeinflussung zwecks der extrakorporalen Bereitstellung eines von dem ersten Übertragungsglied im wesentlichen unbeeinflussten Messsignals und zur Nachbildung der durch das zweite Übertragungsglied verursachten Signalbeeinflussung des Messsignals zwecks extrakorporaler Nachbildung des Ausgangssignals des zweiten Übertragungsglieds, insbesondere zur externen Modellierung des Verhaltens des implantierbaren Geräts in Abhängigkeit von dem Messsignal.

Der Aufbau eines modernen, mittels eines Programmiergerätes auf die individuellen Bedingungen eines Patienten anpaßbaren medizinischen Geräts gestattet es üblicherweise, Signale aus dem Körperinneren aufzunehmen. Dabei kann es sich einerseits um Signale handeln, welche als solche möglichst unverfälscht vom Meßort übertragen werden sollen, oder aber um Signale, welche das Betriebsverhalten des implantierbaren Geräts beeinflussen. Diese Signale werden durch Signalübertragungsmittel beeinflußt, welche innerhalb des Verarbeitungsteils des implantierbaren Geräts vorgesehen und der Telemetriestufe vor- bzw. nachgeschaltet sind.

So ist bei einem modernen Herzschrittmacher in der Regel die Übertragung der elektrischen Aktionspotentiale des Herzens in Form des intrakardialen Elektrokardiogramms an ein extrakorporales Gerät zum Zwecke der Auswertung und/oder Aufzeichnung vorgesehen.

Die in dem intrakardialen EKG repräsentierten Aktionssignale des Herzens werden durch ein für den normalen Betrieb des Herzschrittmachers vorgesehenes Eingangsfilter aufgenommen und danach weiterverarbeitet. Aufgrund der Übertragungseigenschaften des Eingangsfilters des Herzschrittmachers, insbesondere durch den Frequenzgang des Filters, werden die Aktionssignale des Herzens in erheblichem Maße verändert, so daß sie bei der Auswertung in einem EKG-Analysengerät keine sichere Diagnose zulassen.

Aus DE--A-2 545 802 ist ein Herzsignaldiskriminator bekannt, der zur Unterdrückung von Störsignalen dient, welche gleichzeitig mit dem Herzaktionssignal erfaßt werden. Der Herzsignaldiskriminator weist Mittel auf, durch die die erfaßten Signale gleichgerichtet und gleichzeitig bedämpft werden.

Aus EP-A- 0 012 709 ist weiterhin eine digital gesteuerte Amplitudenregeleinrichtung für elektrokardiographische Signale bekannt. Die vorgeschlagene Regeleinrichtung weist Mittel auf, welche den Übertragungsfaktor jeweils auf das Erscheinen eines Impulssignals (QRS-Komplex) hin nach Vergleich der Signalamplitude mit einem Schwellwert jeweils um eine Stufe vergrößern oder verkleinern.

Die aus den genannten Druckschriften bekannten Schaltungsanordnungen beziehen sich im wesentlichen auf eine Anpassung der Amplitude der intrakardialen Signale, um Störsignale in ihrer Wirksamkeit zumindest einzuschränken. Dies ist erforderlich, da die Amplitude dieser sogenannten Artefakte das kardiale Nutzsignal häufig um das Hundertfache übertrifft.

In DD-A-295 995 wird eine Schaltungsanordnung zur Eliminierung von Herzschrittmacherimpulsanteilen aus EKG-Ableitungen beschrieben, mit der (Oberflächen-)EKG-Signale duch geeignete Filterung am Ausgang des EKG-Verstärkers von Schrittmacherimpulsanteilen befreit werden können.

DE-A-3 701 947 beschreibt ein Verfahren zur Erfassung eines Oberflächen-EKG, bei dem eine zuverlässige Verarbeitung der Herzaktionssignale auch bei Schrittmacherträgern dadurch gesichert werden soll, daß die Betriebsparameter des Schrittmachers mittels einer drahtlosen Signalübertragungseinrichtung ausgelesen und dann bei der Verarbeitung des Oberflächen-EKG berücksichtigt werden.

In US-A-5 421 830 wird ein Programmierungssystem für einen Schrittmacher oder Kardioverter/Defibrillator mit Mitteln zur Aufzeichnung und Analyse eines intrakardial aufgenommenen Herzsignals beschrieben, bei dem neben Mitteln zur Speicherung und Wiedergabe der Herzsignale auch eine Simulation des Ansprechens des implantierten Gerätes auf diese vorgesehen ist.

Aus der DE 34 40 615 C1 ist ein Verfahren zum Übertragen von Tonsignalen bekannt, bei welchem zur Anpassung an die Bandbreite eines Übertragungskanals eine Bitratenreduktion unter Zuhilfenahme zweier digitaler Filter vorgenommen wird.

Aus der US 5421 830 ist ein Programmiersystem für einen Herzschrittmacher bekannt, welcher eine extrakorporal einsetzbare Programmiereinheit umfasst, mit welchem Herzsignaldaten erfasst und an die Programmiereinheit übertragen werden können, um ein Verhalten des Herzschrittmachers extrakorporal nachzubilden. Extrakorporal gefundene Parameter zur Einstellung des Herzschrittmachers können anschließend an den Herzschrittmacher zur Anwendung an einem Patienten übertragen werden.

Die vorbekannten Schaltungsanordnungen lösen jedoch nicht das Problem, daß das intrakardiale EKG durch das Eingangsfilter des Herzschrittmachers verfälscht wird. Es ist deshalb zwar möglich, das intrakardiale EKG über einen Telemetriesender zur differenzierten Auswertung bzw. Speicherung an ein extrakorporales Kontrollgerät zu übertragen. Der diagnostische Wert dieses intrakardialen EKGs wie auch seine Brauchbarkeit für eine etwaige Simulation des Ansprechens des Herzschrittmachers ist jedoch durch die signalbeeinflussende Wirkung des in dem Herzschrittmacher angeordneten Eingangsfilters bzw. ggfs. anderer Übertragungsglieder stark herabgesetzt.

Darüberhinaus besteht bei den vorbekannten Schaltungsanordnungen das Problem, daß das intrakardiale EKG stromabwärts des Punktes, an dem es zur Zuführung an die Telemetrieeinheit abgegriffen wurde, durch Verstärker und Filter weiter beeinflußt wird, bevor das auf diese Weise aufbereitete Signal einer Detektoreinrichtung, etwa einem Schwellwertdetektor, bzw. einer Verarbeitungseinheit zugeführt wird. Das an dem extrakorporalen Gerät zur Verfügung stehende Signal unterscheidet sich deshalb von dem im Herzschrittmacher am Eingang der Detektor- bzw. Verarbeitungseinrichtung anliegenden Signal, so daß die Auswertung des telemetrisch übertragenen Signals keine verläßliche Aussage über das Detektions- bzw. Verarbeitungsverhalten des Herzschrittmachers ermöglicht.

Die oben angesprochenen Probleme bestehen auch hinsichtlich der externen Analyse sonstiger Körpersignale, die von einem im Verarbeitungskanal liegenden Abgriffspunkt in einem implantierten medizinischen Gerät nach außen übermittelt werden, so etwa auch für die körperliche Aktivität eines Patienten charakterisierende Signale.

Der Erfindung liegt somit die Aufgabe zugrunde, eine extrakorporale Vorrichtung zur Benutzuung mit einem implantierbaren elektromedizinischen Gerät zu schaffen, die eine möglichst unverfälschte Bereitstellung und ggfs. in Bezug auf die Funktion das implantierten Gerätes aussagekräftige Weiterverarbeitung intrakorporal erfaßter Signale außerhalb des Körpers ermöglicht.

Die Aufgabe wird durch eine Vorrichtung der eingangs genannten Art gelöst, wobei die Extrakorporale Vorrichtung eine dem dritten Übertragungsglied nachgeschaltete Detektoreinrichtung aufweist, welche ein bipolares Schwellwertglied umfasst, welches ausgebildet ist, ein verstärktes und gefiltertes Eingangssignal mit einem positiven und einem negativen Schwellwert zu vergleichen und beim Überschreiten des positiven Schwellwertes beziehungsweise beim Unterschreiten des negativen Schwellwertes einen Herzschlag zu detektieren. Die Detektoreinrichtung ist zusätzlich ausgebildet, die zeitliche Folge und die Dauer der Schwellwertüberschreitungen auszuwerten.

Die Erfindung schließt die technische Lehre ein, bei der extrakorporalen Vorrichtung zum einen die durch ein vor dem Abgriffspunkt des Signals liegendes Übertragungsglied - etwa das Eingangsfilter eines Herzschrittmachers - verursachte Signalbeeinflussung zu kompensieren, um ein unverfälschtes Signal zu erhalten, und ggfs. zum anderen die in dem implantierten Gerät zwischen dem Signalabgriffspunkt und dem eigentlichen Detektor bzw. der Verarbeitungseinheit erfolgende Signalbeeinflussung durch Verstärker und Filter zu simulieren, um extern das gesamte Detektionsverhalten des implantierten Gerätes nachbilden zu können.

Der Begriff "Eingangssignal" ist im folgenden in einem allgemeinen Sinne zu verstehen: Besonders vorteilhaft ist die Erfindung auf natürliche (spontane) Herzaktionssignale, d.h. bei der externen Verarbeitung intrakardialer EKGs, anwendbar. Die Anwendung der Erfindung ist jedoch auch bei Körpersignalen mit Vorteil möglich, die die Aktivität des Herzschrittmacherträgers widerspiegeln, wie beispielsweise intrathorakal oder intrakardial erfaßten Impedanzsignalen. Sie ist jedoch hierauf nicht beschränkt, sondern prinzipiell für alle aus dem internen Verarbeitungskanal eines implantierten Gerätes nach außen übermittelten Signale anwendbar.

Bei einem Herzschrittmacher ist das erste in dem Eingangskanal angeordnete Übertragungsglied in der Regel ein Eingangsfilter, das beispielsweise als Anti-Aliasing-Filter ausgeführt ist.

Das zweite Übertragungsglied besteht in der Regel aus einem Verstärker mit programmierbarer Verstärkung, der durch eine Einstellung der Verstärkung die Anpassung des Signaldetektors an den elektrischen Pegel des Eingangssignals ermöglicht, bzw. einer entsprechend programmierbaren Veratärker-Filter-Baugruppe.

Die Übertragungsglieder können auch jeweils aus mehreren einzelnen Bauelementen bestehen.

Herzschrittmacher, mit denen Ausführungen des erfindungsgemäßen Gerätes vorteilhaft einsetzbar sind, weisen zur Übertragung des Körpersignals (speziell inrakardialen EKGs) nach außerhalb des Körpers einen Telemetriesender auf, der vor dem zweiten Übertragungsglied und/oder nach dem ersten Übertragungsglied mit dem Verarbeitungsskanal verbunden ist. In der Regel ist dieser Telemetriesender Bestandteil einer Telemetrieeinheit des Schrittmachers, die sowohl Sende- als auch Empfangsbetrieb erlaubt, wobei im Sendebetrieb eine Übertragung der von dem Herzschrittmacher aufgenommenen Signale erfolgt, während der Empfangsbetrieb eine Programmierung des Herzschrittmachers durch das extrakorporale Kontrollgerät ermöglicht.

Wenn der Schrittmacher nur ein Übertragungsglied aufweist, erfolgt der Signalabgriff für den Telemetriesender also entweder stromaufwärts oder stromabwärts dieses Übertragungsgliedes.

Weist der Herzschrittmacher dagegen zwei oder mehrere Übertragungsglieder auf (was in der Praxis die Regel ist), so erfolgt der Signalabgriff zwischen den Übertragungsgliedern.

Die erfindungsgemäße Vorrichtung weist in einer auf implantierte Geräte mit Signalabgriff nach einem Übertragungsglied zugeschnittenen Ausführung, dem Telemetrieempfänger nachgeschaltet, ein drittes Übertragungsglied auf, das die Aufgabe hat, die Signalbeeinflussung durch das dem Telemetriesender vorgeschaltete erste Übertragungsglied - in der Regel das Eingangsfilter - zu kompensieren, um das unverfälschte Eingangssignal zur Verfügung zu stellen. Dies ist besonders vorteilhaft bei der Aufnahme und Übertragung eines intrakardialen EKGs als Eingangssignal, da ein unverfälschtes EKG für den behandelnden Arzt einen wesentlich größeren diagnostischen Wert hat, als die mit bekannten Kontrollgeräten ermittelten, durch das Eingangsfilter des Herzschrittmachers verzerrten EKG-Signale. Das dritte Übertragungsglied weist deshalb insbesondere eine gegenüber dem ersten Übertragungsglied exakt inverse Übertragungsfunktion auf, um dessen signalbeeinflussende Wirkung möglichst vollständig kompensieren zu können.

Weist das erste Übertragungsglied im Herzschrittmacher beispielsweise die komplexe Übertragungsfunktion $\underline{c}_1(f)$ auf, so berechnet sich das Telemetriesignal $\underline{TS}(f)$ aus dem Eingangssignal $\underline{ES}(f)$ nach der Formel:

$$\underline{TS}(f) = \underline{c}_1(f) \cdot \underline{ES}(f)$$

Das Ausgangssignal $\underline{AS}(f)$ des dritten Übertragungsglieds mit der komplexen Übertragungsfunktion $\underline{c}_3(f)$ errechnet sich dann aus dem Telemetriesignal $\underline{TS}(f)$:

$$\underline{AS}(f) = \underline{c}_3(f) \cdot \underline{TS}(f)$$

Die Übertragungsfunktion $\underline{c}_3(f)$ muß hierbei so gewählt werden, daß das Ausgangssignal $\underline{TS}(f)$ des dritten Übertragungsglieds das Eingangssignal möglichst unverfälscht wiedergibt. Hieraus ergibt sich die Forderung:

$$\underline{c}_3(f) = 1 / \underline{c}_1(f)$$

Bei implantierten Geräten mit Signalabgriff vor weiteren Übertragungsgliedern unterscheidet sich das am Ausgang der stromabwärts vom Abgriffspunkt vorgesehenen Übertragungsglieder (hier als "zweites Übertragungsglied" subsumiert) erscheinende Signal von dem telemetrisch übertragenen Signal. Eine Modellierung des Schrittmacherverhaltens wird aber dennoch dadurch ermöglicht, daß die Übertragungsfunkber dennoch dadurch ermöglicht, daß die Übertragungsfunktion dieses Gliedes auch im externen Gerät auf das dort vorliegende Signal angewandt wird. Das extern angeordnete vierte Übertragungsglied (unter diesen Begriff werden hier wiederum ggfs. mehrere in der extrakorporalen Vorrichtung vorgesehene Übertragungsglieder gefaßt) weist deshalb insbesonere exakt die gleiche Übertragungsfunktion wie das hinter dem Telemetriesender angeordnete zweite Übertragungsglied auf.

In einer praktisch besonders bedeutsamen Fortbildung dieser Variante ermöglicht das Vorsehen von Mitteln zur Einstellung verschiedener Übertragungsfunktionen des vierten Übertragungsgliedes eine Modellierung des Schrittmacherverhaltens für verschiedene Einstellungen des zweiten Übertragungsglieds, ohne den Herzschrittmacher selbst umprogrammieren zu müssen.

Wie bereits vorstehend erläutert, weist das in dem Herzschrittmacher angeordnete zweite Übertragungsglied in der Regel einen programmierbaren Verstärker auf, der eine Anpassung der nachgeschalteten Detektor- bzw. Verarbeitungseinrichtung an den Pegel der erfaßten Herzsignale ermöglicht. Dies ist wichtig, da der Pegel der von dem Herzschrittmacher erfaßten Herzsignale von der Lage der Elektroden, dem Widerstand des Körpergewebes und anderen Faktoren abhängt und somit Schwankungen unterliegt, die durch eine Einstellung der Eingangsverstärkung berücksichtigt werden müssen.

Bei vorbekannten Schrittmachersystemen erfolgt diese Einstellung in der Regel, indem der behandelnde Arzt nacheinander verschiedene Eingangsverstärkungen programmiert und anhand von Sense-Markern oder aufgrund des Schrittmacherverhaltens beurteilt, ob der Schrittmacher ein vom Herz geliefertes Signal erkennt oder nicht ("Sensingtest"). Problematisch ist hierbei, daß sich die während des Tests vorgenommenen Einstellungen der Eingangsverstärkung bereits auf das Schrittmacherverhalten auswirken, was während des Tests oftmals unerwünscht ist. So werden bei zu hoher Eingangsempfindlichkeit bereits Störsignale fehlerhaft als Herzaktionen detektiert, so daß während des Tests eventuell eine an sich erforderliche Stimulation ausbleibt. Ist die Eingangsempfindlichkeit dagegen zu gering, so werden die spontanen Herzaktionen nicht detektiert, und der Herzschrittmacher stimuliert während des Tests ständig, obwohl das Herz selbst hinreichend schnell schlägt.

Ein weiterer Nachteil der vorbekannten Schrittmachersysteme ist darin zu sehen, daß der Arzt bei der Programmierung des Schrittmachers in der Regel eine höhere Eingangsempfindlichkeit einstellt als tatsächlich erforderlich ist, um auch bei Intensitätsschwankungen der Herzsignale ein Herzereignis sicher erkennen zu können. Dies führt jedoch dazu, daß der Eingangsverstärker des Herzschrittmachers bei einem Herzereignis mit normaler Intensität in die Sättigung gerät, so daß die Amplitude dieses Herzereignisses nicht mehr bestimmt werden kann.

In einer vorteilhaften Variante der Erfindung ist dem vierten Übertragungsglied deshalb eine Detektoreinrichtung nachgeschaltet, die im wesentlichen das gleiche Detektionsverhalten aufweist wie diejenige des Schrittmachers. Das Übertragungsverhalten des vierten Übertragungsglieds ist hierbei einstellbar, um die Übertragungsfunktion zu ermitteln, die ein optimales Detektionsverhalten ermöglicht. Da die Einstellung hierbei in der extrakorporalen Vorrichtung erfolgt und nicht - wie bei bekannten Schrittmachersystemen - im Schrittmacher selbst, wird das Schrittmacherverhalten während des Tests nicht beeinflußt. Erst nach Ermittlung der optimalen Einstellung wird diese vom externen Gerät telemetrisch an den programmierbaren Herzschrittmacher übertragen und eingestellt. Vorzugsweise besteht das vierte Übertragungsglied hierbei aus einem digitalen Filter, dessen Filtercharakteristik durch Vorgabe mehrerer Filterkoeffizienten einstellbar ist.

Die extrakorporale Vorrichtung kann in zweckmäßigen Ausführungen als spezielles Kontrollgerät für ein implantiertes Gerät (insbesondere einen Herzschrittmacher, Defibrillator und/oder Kardioverter), als Baugruppe eines Programmiergerätes für derartige implantierte Geräte oder eines Körpersignal-Analysengerätes (etwa eines EKG- oder EEG-Gerätes) oder auch als - insbesondere programmierbare - Schnittstelle ausgebildet sein, über die ein herkömmliches Programmier- oder Analysegerät auf wesentlich funktionserweiternde Weise mit einem implantierten Gerät verbindbar ist.

Andere vorteilhafte Weiterbildungen der Erfindung sind in den Unteransprüchen gekennzeichnet bzw. werden nachstehend zusammen mit der Beschreibung der bevorzugten Ausführung der Erfindung anhand der Figuren näher dargestellt. Es zeigen:

Figur 1 als bevorzugtes Ausführungsbeispiel der Erfindung ein extrakorporales Kontrollgerät für einen implantierbaren Schrittmacher als Blockschaltbild,

Figur 2a einen exemplarischen Signalverlauf für das in dem Herzschrittmacher aufbereitete intrakardiale Herzsignal sowie

Figur 2b das entsprechende Signal in dem extrakorporalen Kontrollgerät nach Fig. 1 in digitalisierter Form.

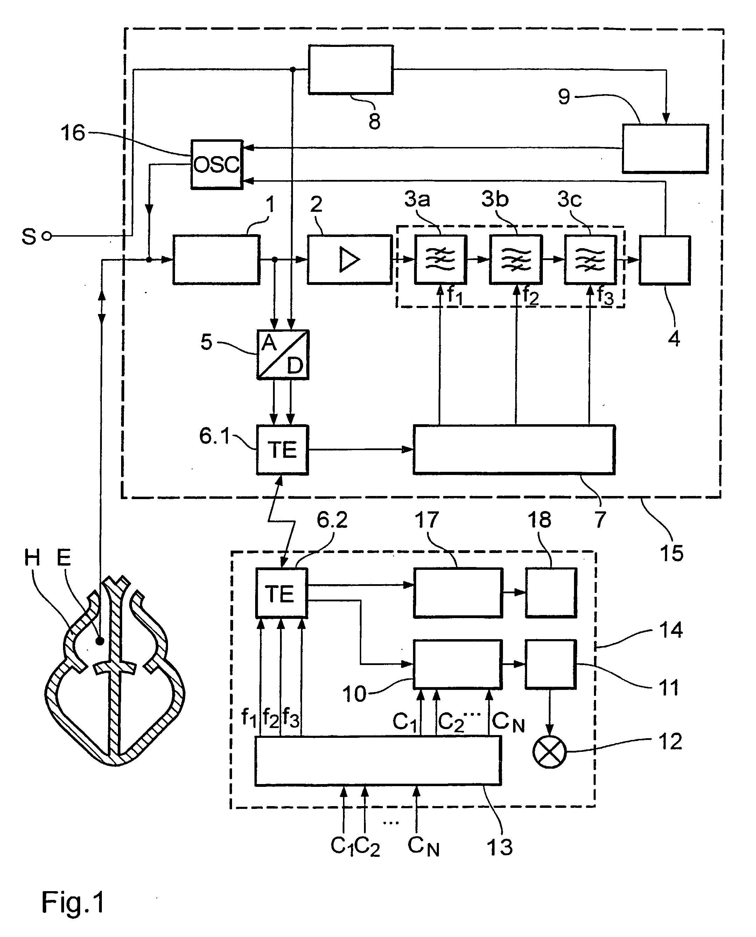

Das in Figur 1 gezeigte Blockschaltbild verdeutlicht den Aufbau eines extrakorporalen Kontrollgeräts 14 im Zusammenwirken mit einem programmierbaren Herzschrittmacher 15. Von beiden Geräten sind nur die in Bezug zur Erfindung stehenden Baugruppen dargestellt; speziell die herkömmlichen übrigen Baugruppen eines Schrittmachers werden als bekannt vorausgesetzt und sind daher hier nicht gezeigt.

Der dargestellte Herzschrittmacher 15 arbeitet nach dem Demand-Prinzip, d.h. er gibt Stimulationsimpulse nur dann an das Herz ab, wenn dieses selbst nicht oder nicht hinreichend schnell schlägt. Zur Detektion von Herzsignalen weist der Herzschrittmacher 15 deshalb eine mit dem Herzgewebe H verbundene Elektrode E auf, die zur Aufbereitung des Eingangssignals mit einem Anti-Aliasing-Filter 1 verbunden ist. Neben der Erfassung der spontanen Herzaktivität dient diese Elektrode zur Abgabe von Stimulationsimpulsen und ist deshalb zusätzlich mit dem Ausgang eines Impulsgenerators 16 verbunden.

Das Ausgangssignal des Anti-Aliasing-Filters wird einem Verstärker 2 zugeführt, der das Eingangssignal um den Faktor 32 verstärkt.

Dem Verstärker 2 ist ein Übertragungsglied 3 mit mehreren Filterelementen 3a, 3b, 3c mit einstellbaren Grenzfrequenzen $f_1$, $f_2$, $f_2$ nachgeschaltet, das durch Einstellung der Grenzfrequenzen $f_1$, $f_2$, $f_3$ eine Anpassung des intrakardial abgenommenen Eingangssignals an eine nachgeschaltete Detektoreinrichtung 4 ermöglicht. Hierdurch können Störsignale ausgefiltert werden, die zusammen mit den Herzsignalen über die Elektrode aufgenommen werden. Zur Einstellung der Filtercharakteristik weist das Übertragungsglied 3 mehrere Steuereingänge auf, über die die Grenzfrequenzen der einzelnen Filterelemente 3a, 3b, 3c eingestellt werden können.

Die dem Übertragungsglied 3 nachgeschaltete Detektoreinrichtung 4 umfaßt als Hauptbestandteil ein bipolares Schwellwertglied, das das verstärkte und gefilterte Eingangssignal mit einem positiven und einem negativen Schwellwert vergleicht und beim Überschreiten des positiven Schwellwerts bzw. beim Unterschreiten des negativen Schwellwerts unter bestimmten Umständen einen natürlichen Herzschlag detektiert. Der Signaldetektor 4 wertet hierbei zusätzlich die zeitliche Folge und die Dauer der Schwellwertüberschreitungen aus, um bei einer Verschiebung des Signalpegels, beispielsweise aufgrund einer Elektrodenverschiebung im Körper des Herzschrittmacherträgers mit der Folge einer dauerhaften Über- oder Unterschreitung eines Schwellwerts, eine Fehldetektion zu verhindern.

Wenn die Detektoreinrichtung 4 auf die vorstehend beschriebene Weise einen natürlichen Herzschlag detektiert, führt dies zu einer Inhibierung des Impulsgenerators 16, der ansonsten mit vorgegebener Stimulationsfrequenz (siehe unten) stimuliert. Dies bedeutet, daß der Impulsgenerator 16 seinen internen Taktgeber zurücksetzt und somit erst dann einen Stimulationsimpuls abgibt, wenn innerhalb einer vorgegebenen Zeitspanne nach der Detektion kein weiterer natürlicher Herzschlag detektiert wird.

Zur Festlegung der Stimulationsfrequenz entsprechend dem aktuellen hämodynamischen Bedarf des Patienten dient ein - hier außerhalb des Herzens dargestellter - Körperfühler S (der beispielsweise in an sich bekannter Weise als intrathorakal angeordnete Impedanzmeßsonde ausgeführt sein kann). Dessen Meßsignal wird einer integrierten Verstärker- und Filterbaugruppe 8 zugeführt, wo eine Signalaufbereitung erfolgt, und gelangt von dort zu einer Verarbeitungseinheit 9, die aus dem aufbereiteten Körpersignal - ebenfalls in bekannter Weise - die Stimulationsrate des Schrittmachers errechnet und den Impulsgenerator 16 entsprechend steuert.

Neben der vorstehend beschriebenen herkömmlichen Funktionsweise als ratenadaptiver Demand-Schrittmacher ermöglicht der Herzschrittmacher 15 die Übertragung eines intrakardialen Elektrokardiogramms (auch als "IECG" bezeichnet) sowie der Signale des Körperfühlers S an ein extrakorporales Kontrollgerät 14.

Stromabwärts des Anti-Aliasing-Filters 1 ist deshalb ein zweikanaliger Analog/Digital-Wandler 5 an den Eingangskanal angeschlossen, der das gefilterte Eingangssignal des Herzschrittmachers sowie das Meßsignal des Körperfühlers S zunächst in ein digitales Datenwort umwandelt, was eine anschließende digitale Datenübertragung an das extrakorporale Kontrollgerät 14 ermöglicht.

Hierzu weist der Schrittmacher 15 eine Telemetrieeinheit 6.1 auf, die sowohl Sende- als auch Empfangsbetrieb ermöglicht. Im Sendebetrieb wird das über die Elektrode E aufgenommene IECG und/oder das Signal des Körperfühlers S an das extrakorporale Kontrollgerät 14 übertragen, während im Empfangsbetrieb Programmiersignale zur Einstellung der Betriebsparameter des Herzschrittmachers 15 empfangen werden.

Eine Programmiermöglichkeit betrifft hierbei die Übertragungsfunktion des Übertragungsglieds 3, was eine externe Anpassung der Detektoreinrichtung 4 an das über die Elektrode aufgenommene Eingangssignal ermöglicht, indem die gewünschte Übertragungsfunktion programmiert wird.

Entsprechend weist auch das extrakorporale Kontrollgerät 14 eine Telemetrieeinheit 6.2 auf, die den Empfang des IECG sowie - im Sendebetrieb - die Programmierung des Herzschrittmachers 15 ermöglicht.

Weiterhin verfügt das extrakorporale Kontrollgerät 14 über ein rekursives Digitalfiltersystem 10 zweiter Ordnung, das eine Filterung des intrakardialen EKG-Signals mit variabler Filtercharakteristik ermöglicht. Das digitale Filter 10 berechnet hierbei aus dem als Folge $x_1$, $x_2$, ...., $x_i$, ... digitaler Abtastwerte vorliegenden intrakardialen EKG-Signal als Ausgangssignal eine Folge $y_1$, $y_2$, ..., $y_i$ nach der Formel

$$y(n) = \sum_{k=1}^{N} c_k \cdot y(n-k) + \sum_{k=0}^{M} c_{N+1+k} \cdot x(n-k); \quad M \leq N$$

Die Übertragungsfunktion dieses Filters 10 errechnet sich wie folgt:

$$U(f) = \sqrt{\frac{\left(\sum_{i=1}^{N} c_i \cdot \cos(2 \cdot \pi \cdot f \cdot (i-1))\right)^2 + \left(\sum_{i=1}^{N} c_i \cdot \sin(2 \cdot \pi \cdot f \cdot (i-1))\right)^2}{1 - \left(\sum_{i=N+1}^{M} c_i \cdot \cos(2 \cdot \pi \cdot f \cdot i)\right)^2 + \left(\sum_{i=N+1}^{M} c_i \cdot \sin(2 \cdot \pi \cdot f \cdot i)\right)^2}}$$

Durch Einstellung der Filterkoeffizienten $c_i$ läßt sich die Filtercharakteristik in weiten Grenzen verändern.

Das Ausgangssignal dieses digitalen Filters 10 wird innerhalb des Kontrollgeräts 14 einer Detektor- bzw. Verarbeitungseinrichtung 11 zugeführt, die das gleiche Detektionsverhalten aufweist wie die im Schrittmacher 15 befindliche Detektoreinrichtung 4. Auf diese Weise läßt sich das Detektionsverhalten des Herzschrittmachers 15 für verschiedene Filtercharakteristiken modellieren, ohne den Herzschrittmacher selbst jeweils umprogrammieren zu müssen. Das Kontrollgerät 14 weist hierzu eine Steuereinheit 13 auf, in die der behandelnde Arzt die Filterkoeffizienten $c_i$ eingibt und somit die Filtercharakteristik des digitalen Filters 10 bestimmt. Die Steuereinheit 13 programmiert diese Filterkoeffizienten $c_i$ dann in das digitale Filter 10, so daß der behandelnde Arzt anhand des Ausgangssignals der Detektoreinrichtung 11 die Auswirkungen der Filtereinstellungen auf das Detektionsverhalten des Schrittmachers beurteilen kann. Als Mittel zur Überprüfung des Detektionsverhaltens ist in der Figur eine Anzeigelampe 12 dargestellt, die jeweils die Erfassung einer Herzaktion anzeigt. Selbstverständlich wird in der Praxis eine verfeinerte Aufbereitung und auch Speicherung des jeweiligen Detektionsergebnisses erfolgen, um die Wirkung verschiedener Filtereinstellungen vergleichen zu können.

Neben der einstellbaren Digitalfilterbaugruppe 10 ist mit dem Ausgang der zweiten Telemetrieeinheit 6.2 eine Filter- und Verstärkereinheit 17 verbunden, der von der Telemetrieeinheit das (bei der Übertragung durch Codierung gekennzeichnete) Signals des Körperfühlers S zugeführt wird. Deren Übertragungskurve entspricht derjenigen der Baugruppe 8 im Schrittmacher 15, so daß auch die Beeinflussung des Körper-(z.B. Impedanz-)signals in dessen Signalaufbereitungsweg extern nachgebildet werden kann. Mit dem Ausgang der Filter- und Verstärkereinheit 17 ist eine weitere Anzeigeeinheit 18 verbunden, auf der das dem Eingangssignal der Verarbeitungseinheit 9 des Schrittmachers entsprechende Signal extern dargestellt werden kann. Mittel zur Einstellung der Übertragungsparameter der Baugruppe 8 sind in der Figur nicht gezeigt, können aber auf ähnliche Weise vorgesehen sein und genutzt werden, wie dies oben für das Filter 10 beschrieben wurde.

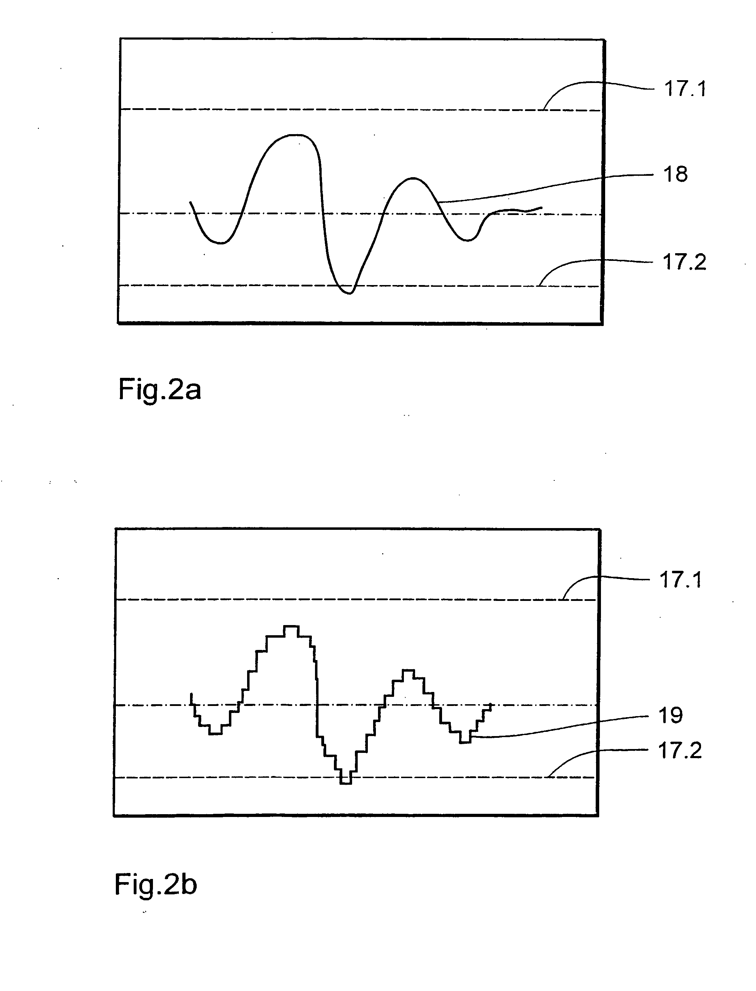

Ein exemplarischer Signalverlauf für das Ausgangssignal 19 des digitalen Filters 10 ist in Figur 2b dargestellt. Die Schwellwerte des nachgeschalteten Schwellwertglieds 11 sind hierbei durch waagerechte gestrichelte Linien 17.1, 17.2 dargestellt. Bei diesem Signalverlauf detektiert das Schwellwertglied 11 ein Herzereignis, da der untere Schwellwert 17.2 unterschritten wird.

Wenn der Arzt auf diese Weise die optimale Filtercharakteristik gefunden hat, errechnet die Steuereinheit 13 daraus, welche Werte der Grenzfrequenzen $f_1$, $f_2$, $f_3$ zu programmieren sind, damit das in dem Herzschrittmacher 15 befindliche zweite Übertragungsglied 3 diese zuvor ermittelte Filtercharakteristik optimal nachbildet.

Die auf diese Weise ermittelten Werte werden dann von der Telemetrieeinheit 6.2 an eine in dem Herzschrittmacher 15 befindliche Steuereinheit 7 übertragen, die die drei Filterelemente 3a, 3b, 3c entsprechend einstellt, so daß der Herzschrittmacher 15 ein optimales Detektionsverhalten zeigt. Nach der Programmierung dieser Grenzfrequenzen $f_1$, $f_2$, $f_3$ zeigt das Ausgangssignal des zweiten Übertragungsglieds 3 dann den in Figur 2a gezeigten Verlauf 18, der bis auf die fehlende Diskretisierung mit dem in Figur 2b dargestellten, durch das extrakorporale Kontrollgerät 14 simulierten Verlauf übereinstimmt.

Die Erfindung beschränkt sich in ihrer Ausführung nicht auf die vorstehend angegebenen bevorzugten Ausführungsbeispiele. Vielmehr ist eine Anzahl von Varianten denkbar, welche von der dargestellten Lösung auch bei grundsätzlich anders gearteten Ausführungen Gebrauch macht.

**Patentansprüche**

1. System mit einem implantierbaren elektromedizinischen Gerät (15), welches einen Verarbeitungskanal (1 bis 3, 8) zur Verarbeitung und Auswertung wenigstens eines die Funktion des implantierbaren elektromedizinischen Gerätes (15) selbst beeinflussenden, über einen intrakorporalen Signalaufnehmer (E, S) aufgenommenen Messsignals aufweist, in dem ein erstes (1, 5) und ein zweites signalbeeinflussendes Übertragungsglied (2, 3, 8) vorgesehen ist sind,
sowie einer extrakorporalen Vorrichtung (14) zur Benutzung mit dem implantierbaren elektromedizinischen Gerät (15),
wobei das implantierbare elektromedizinische Gerät (15) zum Erzeugen und Übertragen eines von dem Messsignal abgeleiteten Telemetriesignals an die extrakorporale Vorrichtung (14) über eine erste Telemetrieeinheit (6.1) verfügt, deren Eingang mit dem Ausgang des ersten Übertragungsgliedes (1, 5) verbunden ist,
und die extrakorporale Vorrichtung (14) über eine zweite Telemetrieeinheit (6.2) zum Empfang des von dem implantierbaren elektromedizinischen Gerät (15) ausgesandten Telemetriesignals verfügt,
**gekennzeichnet durch**
ein drittes Übertragungsglied (17), dessen Eingang mit dem Ausgang der zweiten Telemetrieeinheit (6.2) und dessen Ausgang mit dem Eingang einer Anzeigeeinheit (18) verbunden ist,
und ein viertes (10) Übertragungsglied, dessen Eingang mit dem Ausgang der zweiten Telemetrieeinheit (6.2) verbunden ist,
wobei das dritte Übertragungsglied (17) ein gegenüber dem Übertragungsverhalten des ersten Übertragungsgliedes (1, 5) inverses Übertragungsverhalten aufweist
und das vierte Übertragungsglied (10) ausgebildet ist, die **durch** das zweite Übertragungsglied (2, 3, 8) verursachte Signalbeeinflussung des Messsignals **durch** ein dem Übertragungsverhalten des zweiten Übertragungsgliedes (2, 3, 8) im wesentlichen entsprechendes Übertragungsverhalten zur externen Modellierung des Verhaltens des implantierbaren elektromedizinischen Gerätes (15) in Abhängigkeit von dem Messsignal nachzubilden.

2. System nach Anspruch 1, **dadurch gekennzeichnet,**
**dass** das implantierbare elektromedizinische Gerät (15) ein Herzschrittmacher, Defibrillator oder Kardioverter ist.

**3.** System nach einem der Ansprüche 1 oder 2, **dadurch gekennzeichnet,**
**dass** die zweite Telemetrieeinheit (6.2) zur Übertragung von Steuerbefehlen an das implantierbare elektromedizinische Gerät (15) zu dessen Programmierung ausgebildet ist.

**4.** System nach Anspruch 3, **dadurch gekennzeichnet,**
**dass** dem vierten Übertragungsglied (10) zur extrakorporalen Verarbeitung des intrakorporal aufgenommenen Messsignals mindestens mittelbar eine Detektoreinrichtung (11) nachgeschaltet ist, die im wesentlichen das gleiche Detektionsverhalten aufweist wie eine entsprechende Detektoreinrichtung (4) des implantierbaren elektromedizinischen Gerätes (15),
**dass** das Übertragungsverhalten des vierten Übertragungsgliedes (10) zur Anpassung des Detektionsverhaltens der nachgeschalteten Detektoreinrichtung (11) veränderbar ist und
**dass** das zweite Übertragungsglied (2, 3, 8) im implantierbaren elektromedizinischen Gerät (15) entsprechend der bei dem vierten Übertragungsglied (10) vorgenommenen Einstellung femsteuerbar ist.

**5.** System nach einem der Ansprüche 1 bis 4, **dadurch gekennzeichnet,**
**dass** das dritte (17) und vierte (10) Übertragungsglied über eine Telemetriestrecke (6.1, 6.2) mittelbar mit einer Elektrode (E) zur Aufnahme eines intrakardialen Herzsignals und/oder einem Körperfühler (S) zur Aufnahme eines nicht-kardialen Körpersignals verbunden ist.

**6.** Extrakorporale Vorrichtung (14), ausgebildet zur Verwendung in einem System nach einem der Ansprüche 1 bis 5, mit wenigstens
einer zweiten Telemetrieeinheit (6.2) zum Empfang eines von einem implantierbaren elektromedizinischen Gerät (15) über eine erste Telemetrieeinheit (6.1) ausgesandten Telemetriesignals,
einem dritten Übertragungsglied (17), dessen Eingang mit dem Ausgang der zweiten Telemetrieeinheit (6.2) und dessen Ausgang mit dem Eingang einer Anzeigeeinheit (18) verbunden ist,
und einem vierten Übertragungsglied (10), dessen Eingang mit dem Ausgang der zweiten Telemetrieeinheit (6.2) verbunden ist,
wobei das implantierbare elektromedizinische Gerät (15) einen Verarbeitungskanal (1 bis 3, 8) zur Verarbeitung und Auswertung wenigstens eines die Funktion des implantierbaren elektromedizinischen Gerätes (15) selbst beeinflussenden, über einen intrakorporalen Signalaufnehmer (E, S) aufgenommenen Messsignals aufweist, in dem ein erstes (1, 5) und ein zweites signalbeeinflussendes Übertragungsglied (2, 3, 8) vorgesehen sind,
**dadurch gekennzeichnet,**
**dass** das dritte Übertragungsglied (17) ein gegenüber dem Übertragungsverhalten des ersten Übertragungsgliedes (1, 5) des implantierbaren elektromedizinischen Gerätes (15) inverses Übertragungsverhalten aufweist und
**dass** das vierte Übertragungsglied (10) ausgebildet ist, die durch das zweite Übertragungsglied (2, 3, 8) des implantierbaren elektromedizinischen Gerätes (15) verursachte Signalbeeinflussung des Messsignals durch ein dem Übertragungsverhalten dieses zweiten Übertragungsgliedes (2, 3, 8) im wesentlichen entsprechendes Übertragungsverhalten zur externen Modellierung des Verhaltens des implantierbaren elektromedizinischen Gerätes (15) in Abhängigkeit von dem Messsignal nachzubilden.

**7.** Extrakorporale Vorrichtung (14) nach Anspruch 6, **dadurch gekennzeichnet,**
**dass** das dritte Übertragungsglied (17) der extrakorporalen Vorrichtung (14) ein zum Übertragungsverhalten eines Eingangsfilters oder eines AD-Umsetzers bzw. einer beliebigen Kombination dieser Funktionseinheiten inverses Übertragungsverhalten aufweist.

**8.** Extrakorporale Vorrichtung (14) nach einem der Ansprüche 6 oder 7, **dadurch gekennzeichnet,**
**dass** das Übertragungsverhalten des dritten Übertragungsgliedes (17) einstellbar, ist.

**9.** Extrakorporale Vorrichtung (14) nach einem der Ansprüche 6 bis 8, **dadurch gekennzeichnet,**
**dass** das Übertragungsverhalten des vierten Übertragungsgliedes (10) einstellbar, ist.

**10.** Extrakorporale Vorrichtung (14) nach einem der Ansprüche 6 bis 9, **dadurch gekennzeichnet,**
**dass** das dritte Übertragungsglied (17) als digitales Filter ausgebildet ist, wobei die Filtercharakteristik durch mehrere Filterkoeffizienten ($c_1$, ..., $c_N$) einstellbar ist.

**11.** Extrakorporale Vorrichtung (14) nach einem der Ansprüche 6 bis 10, **dadurch gekennzeichnet,**
**dass** das vierte Übertragungsglied (10) als digitales Filter ausgebildet ist, wobei die Filtercharakteristik durch meh-

rere Filterkoeffizienten ($c_1$, ..., $c_N$) einstellbar ist.

12. Extrakorporale Vorrichtung (14) nach Anspruch 10, **dadurch gekennzeichnet,**
**dass** das als digitales Filter ausgeführte dritte Übertrapunasglied (17) zur Einstellung der Filterkoeffizienten ($c_1$, ..., $c_N$) mehrere mit Ausgängen einer Steuereinrichtung verbundene Steuereingänge aufweist.

13. Extrakorporale Vorrichtung (14) nach Anspruch 11, **dadurch gekennzeichnet,**
**dass** das als digitales Filter ausgeführte vierte Übertragungsglied (10) zur Einstellung der Filterkoeffizienten ($c_1$, ..., $c_N$) mehrere mit Ausgängen einer Steuereinrichtung (13) verbundene Steuereingänge aufweist.

14. System nach einem der Ansprüche 1 bis 5, **dadurch gekennzeichnet,**
**dass** die extrakorporale Vorrichtung (14) eine extrakorporale Vorrichtung (14) nach einem der Ansprüche 7 bis 13 ist.

**Claims**

1. System comprising an implantable electromedical device (15), which has a processing channel (1 to 3, 8) for processing and evaluating at least one measuring signal influencing the function of the implantable electromedical device (15) itself and received via an intracorporeal signal sensor (E, S), in which a first (1, 5) and a second signal-influencing transmission member (2, 3, 8) are provided, and an extracorporeal apparatus (14) for use with the implantable electromedical device (15), wherein to generate and transmit a telemetry signal derived from the measuring signal to the extracorporeal apparatus (14), the implantable electromedical device (15) has a first telemetry unit (6.1), the input of which is connected to the output of the first transmission member (1, 5), and the extracorporeal apparatus (14) has a second telemetry unit(6.2) for receiving the telemetry signal emitted by the implantable electromedical device (15), **characterised by** a third transmission member (17), the input of which is connected to the output of the second telemetry unit (6.2) and the output of which is connected to the input of a display unit (18), and a fourth (10) transmission member, the input of which is connected to the output of the second telemetry unit (6.2), wherein the third transmission member (17) has a transmission behaviour, which is inverse compared to the transmission behaviour of the first transmission member (1, 5), and the fourth transmission member (10) is configured to emulate the signal influencing of the measuring signal caused by the second transmission member (2, 3, 8) by means of a transmission behaviour substantially corresponding to the transmission behaviour of the second transmission member (2, 3, 8), for external modelling of the behaviour of the implantable electromedical device (15) as a function of the measuring signal.

2. System according to claim 1, **characterised in that** the implantable electromedical device (15) is a cardiac pacemaker, defibrillator or cardioverter.

3. System according to either of claims 1 or 2, **characterised in that** the second telemetry unit (6.2) is configured to transmit control commands to the implantable electomedical device (15) in order to programme it.

4. System according to claim 3, **characterised in that** a detector mechanism (11) is connected downstream, at least indirectly, from the fourth transmission member (10) for extracorporeal processing of the intracorporeally received measuring signal, the detector mechanism having substantially the same detection behaviour as a corresponding detector mechanism (4) of the implantable electromedical device (15), **in that** the transmission behaviour of the fourth transmission member (10) can be changed for adaptation of the detection behaviour of the downstream detection mechanism (11) and **in that** the second transmission member (2, 3, 8) in the implantable electromedical device (15) can be remotely controlled in accordance with the adjustment carried out at the fourth transmission member (10).

5. System according to any one of claims 1 to 4, **characterised in that** the third (17) and fourth (10) transmission member are connected indirectly to an electrode (E) via a telemetry section (6.1, 6.2) to receive an intracardiac heart signal and/or to a body sensor (S) to receive a non-cardiac body signal.

6. Extracorporeal apparatus (14), configured for use in a system according to any one of claims 1 to 5, comprising at least a second telemetry unit (6.2) for receiving a telemetry signal emitted by an implantable electromedical device (15) via a first telemetry unit (6.1), a third transmission member (17), the input of which is connected to the output of the second telemetry unit (6.2) and the output of which is connected to the input of a display unit (18), and a fourth transmission member (10), the input of which is connected to the output of the second telemetry unit (6.2), wherein the implantable electromedical device (15) has a processing channel (1 to 3, 8) for processing and evaluating at least one measuring signal influencing the function of the implantable electromedical device (15) itself and received via an intracorporeal signal sensor (E, S), in which a first (1, 5), and a second signal-influencing transmission member (2, 3, 8) are provided, **characterised in that** the third transmission member (17) has a transmission behaviour which is inverse compared to the transmission behaviour of the first transmission member (1, 5) of the implantable electromedical device (15) and **in that** the fourth transmission member (10) is configured to emulate the signal influencing of the measuring signal caused by the second transmission member (2, 3, 8) of the implantable electromedical device (15) by means of a transmission behaviour substantially corresponding to the transmission behaviour of this second transmission member (2, 3, 8) for external modelling of the behaviour of the implantable electromedical device (15) as a function of the measuring signal.

7. Extracorporeal apparatus (14) according to claim 6, **characterised in that** the third transmission member (17) of the extracorporeal apparatus (14) has a transmission behaviour which is inverse to the transmission behaviour of an input filter or an AD converter or any combination of these functional units.

8. Extracorporeal apparatus (14) according to either of claims 6 or 7, **characterised in that** the transmission behaviour of the third transmission member (17) is adjustable.

9. Extracorporeal apparatus (14) according to any one of claims 6 to 8, **characterised in that** the transmission behaviour of the fourth transmission member (10) is adjustable.

10. Extracorporeal apparatus (14) according to any one of claims 6 to 9, **characterised in that** the third transmission member (17) is configured as a digital filter, the filter characteristic being adjustable by a plurality of filter coefficients $(c_1, ..., c_N)$.

11. Extracorporeal apparatus (14) according to any one of claims 6 to 10, **characterised in that** the fourth transmission member (10) is configured as a digital filter, wherein the filter characteristic can be adjusted by a plurality of filter coefficients $(c_1, ..., c_N)$.

12. Extracorporeal apparatus (14) according to claim 10, **characterised in that** the third transmission member (17) designed as a digital filter has a plurality of control inputs connected to outputs of a control mechanism for adjusting the filter coefficients $(c_1, ..., c_N)$.

13. Extracorporeal apparatus (14) according to claim 11, **characterised in that** the fourth transmission member (10) designed as a digital filter has a plurality of control inputs connected to outputs of a control mechanism (13) for adjusting the filter coefficients $(c_1, ..., c_N)$.

14. System according to any one of claims 1 to 5, **characterised in that** the extracorporeal apparatus (14) is an extracorporeal apparatus (14) according to any one of claims 7 to 13.

**Revendications**

1. Système avec
un appareil électromédical implantable (15), lequel présente un canal de traitement (1 à 3, 8) pour le traitement et l'évaluation d'au moins un signal de mesure influençant automatiquement la fonction de l'appareil électromédical implantable (15), relevé au moyen d'un capteur de signaux intracorporel (E, S), dans lequel sont prévus un premier (1, 5) et un deuxième (2, 3, 8) circuit ou organe de transmission influençant le signal,
ainsi qu'un dispositif extracorporel (14) destiné à être utilisé avec l'appareil électromédical implantable (15),
dans lequel l'appareil électromédical implantable (15) dispose, pour la génération et la transmission d'un signal de télémétrie dérivé du signal de mesure au dispositif extracorporel (14), d'une première unité de télémétrie (6.1) dont l'entrée est reliée à la sortie du premier circuit ou organe de transmission (1, 5),
et le dispositif extracorporel (14) dispose d'une deuxième unité de télémétrie (6.2) pour la réception du signal de télémétrie envoyé par l'appareil électromédical implantable (15),
**caractérisé par**
un troisième circuit ou organe de transmission (17) dont l'entrée est reliée à la sortie de la deuxième unité de télémétrie (6.2) et dont la sortie est reliée à l'entrée d'une unité d'affichage (18),

et un quatrième circuit ou organe de transmission (10) dont l'entrée est reliée à la sortie de la deuxième unité de télémétrie (6.2),

le troisième circuit ou organe de transmission (17) présentant un comportement de transmission inverse par rapport au comportement de transmission du premier circuit ou organe de transmission (1, 5)

et le quatrième circuit ou organe de transmission (10) étant conçu pour simuler l'influence sur le signal de mesure exercée par le deuxième circuit ou organe de transmission (2, 3, 8) par un comportement de transmission correspondant essentiellement au comportement de transmission du deuxième circuit ou organe de transmission (2, 3, 8) pour la modélisation externe du comportement de l'appareil électromédical implantable (15) en fonction du signal de mesure.

2. Système selon la revendication 1, **caractérisé par le fait**
**que** l'appareil électromédical implantable (15) est un stimulateur cardiaque, un défibrillateur ou un cardioverteur.

3. Système selon l'une des revendication 1 ou 2, **caractérisé par le fait**
**que** la deuxième unité de télémétrie (6.2) est conçue pour la transmission d'instructions de commande à l'appareil électromédical implantable (15) pour sa programmation.

4. Système selon la revendication 4, **caractérisé par le fait**
**qu'**un dispositif de détection (11) pour le traitement extracorporel du signal de mesure relevé intracorporellement, qui présente essentiellement le même comportement de détection qu'un dispositif de détection (4) correspondant de l'appareil électromédical implantable (15) est placé au moins indirectement après le quatrième circuit ou organe de transmission (10),
**que** le comportement de transmission du quatrième circuit ou organe de transmission (10) est modifiable pour l'adaptation du comportement de détection du dispositif de détection (11) placé après et
**que** le deuxième circuit ou organe de transmission (2, 3, 8) dans l'appareil électromédical implantable (15) est commandable à distance conformément au réglage effectué sur le quatrième circuit ou organe de transmission (10).

5. Système selon l'une des revendications 1 à 4, **caractérisé par le fait**
**que** le troisième (17) et le quatrième (10) circuits de transmission sont reliés indirectement via une section ou voie de télémétrie (6.1, 6.2) à une électrode (E) pour le relevé d'un signal cardiaque intracardiaque et/ou à une sonde corporelle (S) pour le relevé d'un signal corporel non cardiaque.

6. Dispositif extracorporel (14), conçu pour être utilisé dans un système selon l'une des revendications 1 à 5, avec au moins
une deuxième unité de télémétrie (6.2) pour la réception d'un signal de télémétrie envoyé par un appareil électromédical implantable (15) via une première unité de télémétrie (6.1),
un troisième circuit ou organe de transmission (17) dont l'entrée est reliée à la sortie de la deuxième unité de télémétrie (6.2) et dont la sortie est reliée à l'entrée d'une unité d'affichage (18),
et un quatrième circuit ou organe de transmission (10) dont l'entrée est reliée à la sortie de la deuxième unité de télémétrie (6.2),
l'appareil électromédical implantable (15) présentant un canal de traitement (1 à 3, 8) pour le traitement et l'évaluation d'au moins un signal de mesure influençant automatiquement la fonction de l'appareil électromédical implantable (15), relevé au moyen d'un capteur de signaux intracorporel (E, S), dans lequel sont prévus un premier (1, 5) et un deuxième (2, 3, 8) circuit ou organe de transmission influençant le signal,
**caractérisé par le fait**
**que** le troisième circuit ou organe de transmission (17) présente un comportement de transmission inverse par rapport au comportement de transmission du premier circuit ou organe de transmission (1, 5) de l'appareil électromédical implantable (15)
et
**que** le quatrième circuit ou organe de transmission (10) est conçu pour simuler l'influence sur le signal de mesure exercée par le deuxième circuit ou organe de transmission (2, 3, 8) par un comportement de transmission correspondant essentiellement au comportement de transmission de ce deuxième circuit ou organe de transmission (2, 3, 8) pour la modélisation externe du comportement de l'appareil électromédical implantable (15) en fonction du signal de mesure.

7. Dispositif extracorporel (14) selon la revendication 6, **caractérisé par le fait**
**que** le troisième circuit ou organe de transmission (17) du dispositif extracorporel (14) présente un comportement

EP 0 783 902 B1

de transmission inverse par rapport au comportement de transmission d'un filtre d'entrée ou d'un convertisseur A/N ou encore d'une combinaison quelconque de ces deux unités fonctionnelles.

8. Dispositif extracorporel (14) selon l'une des revendications 6 ou 7, **caractérisé par le fait que** le comportement de transmission du troisième circuit ou organe de transmission (17) est réglable.

9. Dispositif extracorporel (14) selon l'une des revendications 6 à 8, **caractérisé par le fait que** le comportement de transmission du quatrième circuit ou organe de transmission (10) est réglable.

10. Dispositif extracorporel (14) selon l'une des revendications 6 à 9, **caractérisé par le fait que** le troisième circuit ou organe de transmission (17) est conçu comme un filtre numérique, la caractéristique de filtrage étant réglable par plusieurs coefficients de filtrage ($c_1$, ..., $c_N$).

11. Dispositif extracorporel (14) selon l'une des revendications 6 à 10, **caractérisé par le fait que** le quatrième circuit ou organe de transmission (10) est conçu comme un filtre numérique, la caractéristique de filtrage étant réglable par plusieurs coefficients de filtrage ($c_1$, ..., $c_N$).

12. Dispositif extracorporel (14) selon la revendication 10, **caractérisé par le fait que** le troisième circuit ou organe de transmission (17) conçu comme un filtre numérique présente plusieurs entrées de commande reliées aux sorties d'un dispositif de commande pour le réglage des coefficients de filtrage ($c_1$, ..., $c_N$).

13. Dispositif extracorporel (14) selon la revendication 11, **caractérisé par le fait que** le quatrième circuit ou organe de transmission (10) conçu comme un filtre numérique présente plusieurs entrées de commande reliées aux sorties d'un dispositif de commande (13) pour le réglage des coefficients de filtrage ($c_1$, ..., $c_N$).

14. Système selon l'une des revendications 1 à 5, **caractérisé par le fait que** le dispositif extracorporel (14) est un dispositif extracorporel (14) selon l'une des revendications 7 à 13.

Fig.1

17.1

18

17.2

Fig.2a

17.1

19

17.2

Fig.2b